# EUROPEAN PATENT APPLICATION

(11) **EP 4 682 169 A1**
(43) Date of publication of application: **21.01.2026**
(21) Application number: 24770031.3
(22) Date of filing: 15.03.2024
(51) Int. Cl.: C07K 16/28, C07K 19/00, C12N 15/13, C12N 15/62, C12N 15/867, A61K 47/68, A61K 39/395, A61P 35/00

(54) **ANTIBODY SPECIFICALLY BINDING TO CLAUDIN18.2 AND PREPARATION METHOD THEREFOR AND USE THEREOF**

(30) Priority: 16.03.2023 CN 202310259465
(71) Applicant: Gracell Biotechnologies (Shanghai) Co., Ltd., Shanghai 200233 (CN)
(72) Inventor: DONG, Qi, Shanghai 200233 (CN); SHEN, Lianjun, Shanghai 200233 (CN); YIN, Wenjie, Shanghai 200233 (CN); CAO, Wei, Shanghai 200233 (CN)
(74) Representative: Mewburn Ellis LLP
(86) International application number: PCT/CN2024/081935
(87) International publication number: WO 2024/188341

(57) **Abstract**

Provided is an anti-Claudin18.2 nanobody or a binding fragment thereof. The antibody has a good specificity, and a Claudin18.2-targeting immune effector cell prepared using the antibody shows a good therapeutic effect in the treatment or amelioration of diseases having positive expression of Claudin18.2.

## Description

### TECHNICAL FIELD

The present invention relates to the field of engineered immunotherapy, and particularly relates to an antibody that specifically binds to Claudin18.2, and a preparation method therefor and an application thereof.

### BACKGROUND ART

The Claudin family proteins are widely distributed in the tight junction structures of epithelial cells. Claudin18.2 is a membrane protein having four transmembrane regions and two extracellular loop regions, the N-terminus and C-terminus being located in the cytoplasm. The two extracellular regions can serve as specific targets for targeted therapy. In normal tissues, Claudin18.2 is specifically expressed in highly differentiated gastric epithelial cells. In addition to being expressed in gastric cancer, Claudin18.2 is also expressed in tumor cells of bile duct cancer, ovarian cancer, lung cancer, esophageal cancer, pancreatic cancer, etc. in high proportions.

Chimeric antigen receptor T cell (CAR T) is a novel immunotherapy method targeting a specific antigen on the surface of tumor cells. Currently, it is applied to the development of cell therapy for solid tumors.

The advantages of camel-derived antibodies lie in their stable properties, good water solubility, low immunogenicity, stronger affinity, small molecular weight, ability to penetrate into the interior of antigens, and stronger tissue penetration compared to conventional antibodies. Camel-derived antibodies can be used as a diagnostic tool, and can also be used for the delivery of CAR-T and targeting drugs, so as to develop CAR-T having higher affinity, stronger specificity, and lower immunogenicity.

### SUMMARY OF THE INVENTION

An object of the present invention is to provide an antibody that has a high affinity and a high biological activity and can specifically recognize Claudin 18.1 and/or Claudin 18.2 antigens, and an application thereof.

In a first aspect of the present invention, a nanobody that specifically binds to Claudin18.2 is provided. Complementary determining regions, or CDR regions, of a VHH chain of the nanobody are selected from the group consisting of:
(1) CDR1 as shown in SEQ ID NO: 36, CDR2 as shown in SEQ ID NO: 37, and CDR3 as shown in SEQ ID NO: 38;
(2) CDR1 as shown in SEQ ID NO: 39, CDR2 as shown in SEQ ID NO: 40, and CDR3 as shown in SEQ ID NO: 41;
(3) CDR1 as shown in SEQ ID NO: 42, CDR2 as shown in SEQ ID NO: 43, and CDR3 as shown in SEQ ID NO: 44; (4) CDR1 as shown in SEQ ID NO: 45, CDR2 as shown in SEQ ID NO: 46, and CDR3 as shown in SEQ ID NO: 47;
(5) CDR1 as shown in SEQ ID NO: 48, CDR2 as shown in SEQ ID NO: 49, and CDR3 as shown in SEQ ID NO: 50;
(6) CDR1 as shown in SEQ ID NO: 51, CDR2 as shown in SEQ ID NO: 52, and CDR3 as shown in SEQ ID NO: 53;
(7) CDR1 as shown in SEQ ID NO: 39, CDR2 as shown in SEQ ID NO: 54, and CDR3 as shown in SEQ ID NO: 41;
(8) CDR1 as shown in SEQ ID NO: 39, CDR2 as shown in SEQ ID NO: 55, and CDR3 as shown in SEQ ID NO: 41;
(9) CDR1 as shown in SEQ ID NO: 67, CDR2 as shown in SEQ ID NO: 68, and CDR3 as shown in SEQ ID NO: 41;
(10) CDR1 as shown in SEQ ID NO: 39, CDR2 as shown in SEQ ID NO: 69, and CDR3 as shown in SEQ ID NO: 41; and/or
(11) CDR1 as shown in SEQ ID NO: 36, CDR2 as shown in SEQ ID NO: 65, and CDR3 as shown in SEQ ID NO: 66.

In another preferred embodiment, CDR1, CDR2, and CDR3 are separated by framework regions FR1, FR2, FR3, and FR4 of the VHH chain.

In another preferred embodiment, the nanobody that specifically binds to Claudin18.2 includes a humanized antibody, a camel-derived antibody, and a chimeric antibody.

In another preferred embodiment, the amino acid sequence of the nanobody that specifically binds to Claudin18.2 is as shown in any one of SEQ ID NOs: 1-26.

In another preferred embodiment, the amino acid sequence of the VHH chain of the nanobody is selected from the group consisting of: SEQ ID NOs: 1-26, or combinations thereof.

In another preferred embodiment, the CDR regions of the VHH chain of the nanobody comprise an amino acid sequence having at least 80%, preferably at least 90%, more preferably at least 95%, or even more preferably at least 99% sequence identity to any one of SEQ ID NOs: 1-26. In another preferred embodiment, the amino acid sequence of the CDR regions of the VHH chain of the nanobody comprises one or more amino acid substitutions, preferably conservative amino acid substitutions compared with any one of SEQ ID NOs: 1-26.

In another preferred embodiment, any amino acid sequence of the aforementioned amino acid sequences further includes a derivative sequence that optionally comprises addition, deletion, modification and/or substitution of at least one (e.g., 1 to 3, preferably 1 to 2, more preferably 1) amino acid and is capable of retaining the ability of specific binding to Claudin18.2.

In another preferred embodiment, the Claudin18.2 is human or non-human mammal Claudin18.2.

In another preferred embodiment, the Claudin18.2 is human, mouse, rat, or non-human primate (such as monkey) Claudin18.2.

In a second aspect of the present invention, a multivalent antibody or multispecific antibody that specifically binds to Claudin18.2 is provided, the multivalent antibody or multispecific antibody comprising at least one antibody component that targets a Claudin18.2 epitope, the antibody component being the anti-Claudin18.2 nanobody according to the first aspect of the present invention.

In another preferred embodiment, the anti-Claudin18.2 multivalent antibody or multispecific antibody comprises one or more anti-Claudin18.2 nanobodies.

In another preferred embodiment, the anti-Claudin18.2 antibody includes a monobody, a bibody (bivalent antibody), a tetrabody (tetravalent antibody), and/or a multibody (multivalent antibody).

In another preferred embodiment, the anti-Claudin18.2 antibody comprises one or more VHH chains having the amino acid sequence as shown in any one of SEQ ID NOs: 1-26.

In another preferred embodiment, the anti-Claudin18.2 antibody comprises two VHH chains having the amino acid sequence as shown in any one of SEQ ID NOs: 1-26.

In another preferred embodiment, the antibody is selected from: animal derived antibodies, chimeric antibodies, humanized antibodies, or combinations thereof.

In a third aspect of the present invention, a recombinant protein is provided, the recombinant protein comprising:
(i) the nanobody that specifically binds to Claudin18.2 according to the first aspect of the present invention, or the multivalent antibody or multispecific antibody that specifically binds to Claudin18.2 according to the second aspect of the present invention; and
(ii) optionally a tag sequence that aids in expression and/or purification.

In another preferred embodiment, the antibody is a multivalent antibody.

In another preferred embodiment, the tag sequence includes an Fc tag, an HA tag, a Flag tag, and a 6His tag.

In another preferred embodiment, the Fc tag comprises hlgG1Fc and mlgG1Fc.

In another preferred embodiment, the recombinant protein specifically binds to Claudin18.1 and/or Claudin18.2 proteins.

In a fourth aspect of the present invention, a chimeric antigen receptor (CAR) fusion protein is provided, wherein the chimeric antigen receptor (CAR) fusion protein comprises, from N-terminus to C-terminus:
(i) an antigen-binding domain that specifically binds to Claudin18.2, the antigen-binding domain comprising the nanobody according to the first aspect of the present invention;
(ii) a transmembrane domain;
(iii) at least one co-stimulatory domain; and
(iv) an activation domain.

In another preferred embodiment, the antigen-binding domain is monovalent or multivalent.

In another preferred embodiment, the antigen-binding domain is from the nanobody according to the first aspect of the present invention or the recombinant protein according to the third aspect of the present invention.

In another preferred embodiment, the CAR has a structure as shown in Formula la:

L-VHH-H-TM-C-CD3ζ (la)

where,
each "-" is independently a linker peptide or peptide bond;
L is a signal peptide sequence;
VHH is an antigen-binding domain that specifically binds to Claudin18.2;
H is a hinge region;
TM is a transmembrane domain;
C is a co-stimulatory signal domain; and
CD3ζ is a cytoplasmic signal transduction sequence (including wild-type, or mutant/modification thereof) derived from CD3ζ.

In another preferred embodiment, the L is a signal peptide of a protein selected from the group consisting of: CD28, 4-1BB, GM-CSF, CD3, CD8a, or combinations thereof.

In another preferred embodiment, the L is a signal peptide derived from CD8.

In another preferred embodiment, the L comprises an amino acid sequence as shown in SEQ ID NO: 27.

In another preferred embodiment, the amino acid sequence of the VHH is as shown in SEQ ID NO: 2, 5, or 18.

In another preferred embodiment, the H comprises an amino acid sequence as shown in SEQ ID NO: 28.

In another preferred embodiment, the TM includes a transmembrane region derived from CD28.

In another preferred embodiment, the C is a transmembrane region of a protein selected from the group consisting of: CD28, 4-1BB, CD8a, or combinations thereof.

In another preferred embodiment, the C includes a co-stimulatory signal molecule derived from 4-1BB.

In another preferred embodiment, the CAR fusion protein has an amino acid sequence as shown in SEQ ID NOs: 33-35.

In a fifth aspect of the present invention, an antibody-drug conjugate is provided, the antibody-drug conjugate comprising:
(a) the nanobody according to the first aspect of the present invention, the multivalent antibody or multispecific antibody according to the second aspect of the present invention, or the recombinant protein according to the third aspect of the present invention; and
(b) a conjugating moiety that is conjugated to the antibody moiety, the conjugating moiety being selected from the group consisting of: detectable labels, drugs, toxins, cytokines, radionuclides, enzymes, or combinations thereof.

In another preferred embodiment, the antibody moiety is conjugated to the conjugating moiety via a chemical bond or linker.

In a sixth aspect of the present invention, a polynucleotide is provided, the polynucleotide encoding a protein selected from the group consisting of: the nanobody according to the first aspect of the present invention, the multivalent antibody or multispecific antibody according to the second aspect of the present invention, the recombinant protein according to the third aspect of the present invention, or the CAR fusion protein according to the fourth aspect of the present invention.

In a seventh aspect of the present invention, an expression vector is provided, the expression vector comprising the polynucleotide according to the sixth aspect of the present invention.

In another preferred embodiment, the expression vector is selected from the group consisting of: DNA, RNA, viral vectors, plasmids, transposons, other gene transfer systems, or combinations thereof.

In another preferred embodiment, the expression vector is a lentiviral vector.

In an eighth aspect of the present invention, a host cell is provided, the host cell comprising the expression vector according to the seventh aspect of the present invention, or having the polynucleotide as described the sixth aspect of the present invention integrated in a genome thereof, or expressing the nanobody as described the first aspect of the present invention, the multivalent antibody or multispecific antibody as described the second aspect of the present invention, the recombinant protein as described the third aspect of the present invention, or the CAR fusion protein as described the fourth aspect of the present invention.

In another preferred embodiment, the cell is an isolated cell, and/or the cell is a genetically engineered cell.

In another preferred embodiment, the cell is a mammalian cell.

In another preferred embodiment, the cell is a T cell.

In another preferred embodiment, the host cell is an engineered immune cell.

In another preferred embodiment, the engineered immune cell is selected from the group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells); and
(v) chimeric antigen receptor macrophages.

In a ninth aspect of the present invention, a method for preparing an engineered immune cell is provided, comprising the following step of: introducing the nucleic acid molecule according to the sixth aspect of the present invention or the vector according to the seventh aspect of the present invention into a T cell or NK cell, so as to obtain the engineered immune cell, the engineered immune cell expressing the CAR fusion protein according to the fourth aspect of the present invention.

In another preferred embodiment, the method further comprises the step of: determining the function and efficacy of the obtained engineered immune cell.

In a tenth aspect of the present invention, a formulation is provided, the formulation comprising the nanobody according to the first aspect of the present invention, the multivalent antibody or multispecific antibody according to the second aspect of the present invention, the recombinant protein according to the third aspect of the present invention, the CAR fusion protein according to the fourth aspect of the present invention, the vector according to the seventh aspect of the present invention, or the host cell according to the eighth aspect of the present invention, and a pharmaceutically acceptable carrier, diluent or excipient.

In another preferred embodiment, the host cell is an engineered immune cell.

In an eleventh aspect of the present invention, a pharmaceutical composition is provided, the pharmaceutical composition comprising:
(i) the nanobody according to the first aspect of the present invention, the multivalent antibody or multispecific antibody according to the second aspect of the present invention, the recombinant protein according to the third aspect of the present invention, or an immune cell that expresses the CAR fusion protein according to the fourth aspect of the present invention; and
(ii) a pharmaceutically acceptable carrier.

In another preferred embodiment, the conjugating moiety of the immunoconjugate is a drug, a toxin, and/or a therapeutic isotope.

In another preferred embodiment, the pharmaceutical composition further comprises other drugs for treating immune system diseases or tumor diseases.

In another preferred embodiment, the pharmaceutical composition is used to prepare a drug for the prevention and/or treatment of a disease or condition related to Claudin18.2.

In another preferred embodiment, the disease or condition related to Claudin18.2 is selected from the group consisting of: breast cancer, gastric cancer, colorectal cancer, pancreatic cancer, prostate cancer, cervical cancer, head and neck cancer, lung cancer, esophageal cancer, kidney cancer, bladder cancer, uterine cancer, ovarian cancer, or combinations thereof.

In a twelfth aspect of the present invention, a kit is provided, the kit comprising the nucleic acid molecule according to the sixth aspect of the present invention, the recombinant protein according to the third aspect of the present invention, or the vector according to the seventh aspect of the present invention.

In another preferred embodiment, the kit is used to prepare an immune cell that expresses the receptor CAR fusion protein according to the fourth aspect of the present invention.

In a thirteenth aspect of the present invention, provided is a use of the nanobody according to the first aspect of the present invention, the multivalent antibody or multispecific antibody according to the second aspect of the present invention, the recombinant protein according to the third aspect of the present invention, or an immune cell that expresses the CAR fusion protein according to the fourth aspect of the present invention in the preparation of a drug for the prevention and/or treatment of a cancer or tumor related to Claudin18.2.

In a fourteenth aspect of the present invention, a method for treating a disease is provided, comprising administering to a subject in need thereof the nanobody according to the first aspect of the present invention, the multivalent antibody or multispecific antibody according to the second aspect of the present invention, the recombinant protein according to the third aspect of the present invention, or an immune cell that expresses the CAR fusion protein according to the fourth aspect of the present invention.

In another preferred embodiment, the disease is a cancer or tumor having positive expression of Claudin18.2.

It should be understood that within the scope of the present invention, the above-described technical features of the present invention and the technical features specifically described below (as in Examples) can be combined with each other to form new or preferred technical solutions. Due to space limitation, such technical solutions will not be elaborated herein.

### BRIEF DESCRIPTION OF THE DRAWINGS

Fig. 1 shows the binding efficiency of anti-Claudin18.2 recombinant antibodies to 293FT-CLDN18.1 and 293FT-CLDN18.2 cells.
Fig. 2 shows exemplary designs of single CAR and dual epitope CAR of chimeric antigen receptor targeting Claudin 18.2.
Fig. 3 shows the CAR expression level of T cells targeting Claudin 18.2.
Fig. 4 shows the memory phenotypes of CAR-T cells targeting Claudin 18.2 under continuous culture in vitro.
Fig. 5 shows the transient killing effect of CAR-T, the cytotoxicity of CAR-T cells targeting Claudin 18.2 to target cells 293FT-Claudin18.2, 293FT-Claudin18.1, and 293FT cells.
Fig. 6 shows the in vitro cytotoxicity of CAR-T cells targeting Claudin 18.2 to target cells AGS-Claudin18.2.
Fig. 7 shows the levels of cytokine release when CAR-T cells targeting Claudin18.2 were co-cultured with target cells.
Fig. 8 shows a comparison of the in vitro cytotoxicity of humanized Claudin18.2 CAR-T cells and maternal CAR-T cells.
Fig. 9 shows the affinity of binding of the anti-Claudin 18.2 recombinant antibody to Claudin18.2 VLP antigen as determined using ELISA.
Fig. 10 shows the in vivo efficacy of CAR-T cells targeting Claudin18.2.

### DETAILED DESCRIPTION OF PREFERRED EMBODIMENTS

After extensive and in-depth research, the present inventors unexpectedly obtained for the first time an anti-CLDN 18.2 antibody having high and excellent affinity and high anti-tumor activity. Specifically, after extensive screening, the present inventors obtained for the first time multiple nanobodies specifically binding to CLDN18.2. On the basis of the nanobodies, recombinant antibodies and humanized antibodies, as well as Claudin 18.2-targeting chimeric antigen receptors, were further prepared in the present invention. Based on an alpaca heavy chain antibody that specifically binds to Claudin18.2, the present invention provides CAR-Ts having higher affinity, stronger specificity for target antigens, and lower immunogenicity compared to conventional antibodies. The CAR-T cells of the present invention have excellent target cell-killing activity and can well infiltrate into the interior of solid tumors, thereby exhibiting an excellent anti-tumor effect in animals. On that basis, the present invention has been completed.

### TERMS

In order to more easily understand the present disclosure, specific terms are first defined. As used in the present application, unless otherwise expressly indicated herein, each of the following terms shall have the meaning given below. Additional definitions are set forth throughout the application.

The term "about" may refer to a value or composition that is within an acceptable error range for a particular value or composition as determined by one of ordinary skill in the art, which will depend in part on how the value or composition is measured or determined. For example, as used herein, the expression "about 100" includes 99 and 101 and all values therebetween (such as 99.1, 99.2, 99.3, 99.4, etc.).

As used herein, the term "contain" or "include (comprise)" may be open-ended, semi closed-ended, and close-ended. In other words, the term also includes "substantially composed of" or "composed of'.

Sequence identity is determined by comparing two aligned sequences along a predetermined comparison window (the window can be 50%, 60%, 70%, 80%, 90%, 95%, or 100% of the length of a reference nucleotide sequence or protein), and determining the number of positions where the same residue appears. Typically, sequence identity is expressed as a percentage. Methods for measuring sequence identity of nucleotide sequences is well-known for those skilled in the art.

As used herein, the terms "heavy chain variable region" and "VH" can be used interchangeably.

As used herein, the terms "variable region" and "complementary determining region (CDR)" can be used interchangeably.

In the present invention, the term "antibody of the present invention", " protein of the present invention", or "polypeptide of the present invention" can be used interchangeably, and all refer to an antibody that specifically binds to CLDN18.2, such as a protein or polypeptide having a heavy chain variable region (such as an amino acid sequence set forth in SEQ ID NOs: 1-14). They may or may not contain a starting methionine.

### CLDN18.2

Full-length human CLDN18 contains 261 amino acids and is a tetra-transmembrane protein having four transmembrane hydrophobic regions as well as two extracellular loop structures. Loop 1 is formed of transmembrane region 1 and transmembrane region 2 curvingly connected to each other, and loop 2 is formed of transmembrane region 3 and transmembrane region 4 curvingly connected to each other. Human CLDN18.1 and CLDN18.2 differ by eight amino acids in the amino acid composition in N-terminus, transmembrane region 1, and extracellular loop 1, and are identical to each other in the remaining portions. They have a sequence identity of 92%.

### Antibodies

As used herein, the term "antibody" refers to an immunoglobulin, which is a tetrapeptide-chain structure formed of two identical heavy chains and two identical light chains connected by inter-chain disulfide bonds.

Existing antibody numbering schemes include:
1. The Kabat scheme (Kabat et al., 1991) is based on the location of regions of high sequence variation between sequences of the same domain type. Antibody heavy (VH) and light (Vλ and Vk) variable domains are numbered differently.
2. Chothia's scheme (Al-Lazikani, 1997) is the same as Kabat's but corrects where an insertion is annotated around the first VH complementarity determining region (CDR) so that it corresponds to a structural loop. Similarly, the Enhanced Chothia scheme (Abhinandan and Martin, 2008) makes further structural corrections of indel positions.
3. In contrast to these Kabat-like schemes, IMGT (Lefranc, 2003) and AHo (Honegger and Plückthun, 2001) both define unique schemes for antibody and T cell receptor (TCR) (Vα and Vβ) variable domains. Thus, equivalent residue positions can easily be compared between domain types. IMGT and AHo differ in the number of positions they annotate (128 and 149 respectively) and where they consider indels to occur.

Immunoglobulins differ in the composition and arrangement order of the amino acids of the heavy chain constant regions, so their antigenicity is also different. Accordingly, immunoglobulins may be divided into five classes, or immunoglobulin isotypes, namely IgM, IgD, IgG, IgA, and IgE, their corresponding heavy chains being a µ chain, a δ chain, a γ chain, an α chain, and an ε chain, respectively. Each class of Ig may be subdivided into different subclasses according to the differences in the amino acid composition of the hinge region and the number and position of the heavy chain disulfide bond. For example, IgG may be divided into IgG1, IgG2, IgG3, and IgG4. Light chains may be a κ chain or a λ chain depending on the constant region thereof. Each of the five classes of Ig may have a κ chain or a λ chain. The subunit structures and three-dimensional configurations of different classes of immunoglobulin are well-known to those skilled in the art.

The light chains of the antibody according to the present invention may further comprise a light chain constant region, the light chain constant region comprising a human or murine κ chain, λ chain, or variant thereof.

In the present invention, the heavy chains of the antibody according to the present invention may further comprise heavy chain constant regions, the heavy chain constant regions comprising human or murine IgG1, IgG2, IgG3, IgG4 or variants thereof. The sequences of about 110 amino acids towards the N terminus of the heavy chains and the light chains of the antibody vary greatly, and constitute variable regions (Fv regions); the sequences of the other amino acids towards the C terminus are relatively stable, and constitute constant regions. The variable regions include three hypervariable regions (HVRs) and four framework regions (FRs) having a relatively conserved sequence. The three hypervariable regions determine the specificity of the antibody, and are also called complementarity determining regions (CDRs). Each light chain variable region (LCVR) and each heavy chain variable region (HCVR) consist of three CDR regions and four FR regions, which are arranged in the order of FR1, CDR1, FR2, CDR2, FR3, CDR3 and FR4 from the amino terminus to the carboxyl terminus. The three CDR regions of the light chains are designated as LCDR1, LCDR2 and LCDR3, and the three CDR regions of the heavy chains are designated as LHCDR1, HCDR2 and HCDR3. In Example 13 of the present invention, the six CDRs of the ch782 antibody were defined with reference to the Kabat and Chothia methods.

The term "murine antibody" in the present invention refers to an anti-CLDN18.2 monoclonal antibody prepared according to knowledge and skills in the art. During preparation, a test subject is injected with the CLDN18.2 antigen, and a hybridoma expressing the antibody having the desired sequence or functional characteristics is isolated. In a preferred embodiment of the present invention, the murine CLDN18.2 antibody or an antigen-binding fragment thereof may further comprise the light chain constant region of murine κ chain, λ chain, or variant thereof, or may further comprise the heavy chain constant region of murine IgG1, IgG2, IgG3, or variant thereof.

The term "chimeric antibody" refers to an antibody formed by fusing variable regions of a murine antibody with constant regions of a human antibody. The chimeric antibody can alleviate an immune response induced by the murine antibody.

The term "humanized antibody", also known as CDR-grafted antibody, refers to an antibody produced by transplantation of murine CDR sequences into the frameworks of human antibody variable regions, i.e., different types of human germline antibody framework sequences. The humanized antibody can overcome a heterogeneous reaction induced by a chimeric antibody which carries a large amount of murine protein components. Such framework sequences can be obtained from public DNA databases or published references that include germline antibody gene sequences. In order to avoid the decrease in activity resulting from the decrease
of immunogenicity, the human antibody variable region framework sequences can be subjected to minimal reverse mutations or back mutations to maintain the activity.

The term "antigen-binding fragment of an antibody" (or abbreviated "antibody fragment") refers to one or more fragments of an antibody that retain the ability to specifically bind to an antigen (e.g., CLDN18.2). It has been shown that a fragment of a full-length antibody can be used to perform the antigen-binding function of the antibody. Examples of binding fragments included in the term "antigen-binding fragment of an antibody" include
(i) Fab fragment, a monovalent fragment consisting of VL, VH, CL, and CH1 domains;
(ii) F(ab')₂ fragment, a divalent fragment comprising two Fab fragments connected by a disulfide bridge on the hinge region;
(iii) Fd fragment, consisting of VH and CH1 domains; and
(iv) Fv fragment, consisting of the VH and VL domains of a single arm of an antibody.

An Fv antibody comprises a heavy chain variable region and a light chain variable region, but does not comprise a constant region. The Fv antibody is the smallest antibody fragment having all antigen-binding sites. Generally, an Fv antibody further comprises a polypeptide linker between the VH and VL domain, and can form the structure required for antigen binding.

The term "CDR" refers to one of the six hypervariable regions, within the variable domains of an antibody, that mainly contribute to antigen binding. One of the most commonly used definitions of the six CDRs is provided by Kabat E.A et al., (1991) Sequences of proteins of immunological interest. NIH Publication, No. 91-3242

The term "epitope" or "antigenic determinant" refers to a site on an antigen where an immunoglobulin or antibody specifically binds (e.g., a particular site on the CLDN18.2 molecule). An epitope typically comprises at least 3, 4, 5, 6, 7, 8, 9, 10, 11, 12, 13, 14, or 15 consecutive or non-consecutive amino acids in a unique spatial conformation.

The terms "specific binding", "selective binding", "selectively bind", and "specifically bind" refer to binding of an antibody to a predetermined epitope on an antigen. Typically, an antibody binds with an affinity (KD) of approximately
less than 10⁻⁷ M, such as approximately less than 10⁻⁸ M, 10⁻⁹ M or 10¹⁰ M or less.

The term "competitive binding" refers to an antibody that recognizes the same epitope (also referred to as antigenic determinant) or a portion of the same epitope on an extracellular region of CLDN18.2 and binds to the antigen as the monoclonal antibody of the present invention does. An antibody that binds to the same epitope as the monoclonal antibody of the present invention does refers to an antibody that recognizes and binds to an amino acid sequence of CLDN18.2 that is recognized by the monoclonal antibody of the present invention.

The term "KD" or "Kd" refers to the dissociation equilibrium constant of a particular antibody-antigen interaction. Typically, the antibody of the present invention binds to CLDN18.2 with a dissociation equilibrium constant (KD) of less than approximately 10⁻⁷ M, such as less than approximately 10⁻⁸ M, 10⁻⁹ M or 10⁻¹⁰ M or less.

As used herein, the term "antigenic determinant" refers to a three-dimensional spatial site on an antigen that is discontinuous and recognized by the antibody or antigen-binding fragment of the present invention.

The present invention comprises not only the entire antibody, but also a fragment of the antibody or a fusion protein formed by the antibody with an other sequence, the fragment or fusion protein having immune activity. Therefore, the present invention also includes fragments, derivatives, and analogues of the antibody.

In the present invention, the antibody includes murine, chimeric, humanized, or fully human antibodies prepared using techniques familiar to those skilled in the art. A recombinant antibodies, such as a chimeric and humanized monoclonal antibody, comprises both human and non-human portions, and can be prepared using DNA recombination techniques well-known in the art.

As used herein, the term "monoclonal antibody" refers to an antibody secreted by a clone derived from a single cell. The monoclonal antibody is highly specific and targets a single antigenic epitope. The cell may be a eukaryotic, prokaryotic or phage clonal cell line.

In the present invention, the antibody may be monospecific, bispecific, trispecific, or of greater multispecificity.

In the present invention, the antibody of the present invention further includes conserved variants thereof. The conserved variants refer to, compared with the amino acid sequence of the antibody of the present invention, polypeptides formed by replacing at most 10, preferably at most 8, more preferably at most 5, and most preferably 3 amino acids with amino acids having similar or close properties. These conservative variant polypeptides are preferably produced by performing amino acid substitutions according to Table A below.

**Table A**

| Initial residue | Representative substitution(s) | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Humanized anti-CLDN18.2 antibody

The present invention provides a humanized anti-CLDN 18.2 antibody (referred to as CLDN18.2 antibody hereinafter). Specifically, the present invention provides a humanized antibody having high specificity and high affinity to CLDN18.2. The humanized antibody comprises a heavy chain and a light chain, the heavy chain comprising an amino acid sequence of a heavy chain variable region (VH), and the light chain comprising an amino acid sequence of a light chain variable region (VL).

Generally, there are two types of human framework region that could be selected for antibody humanization: one is from a known mature antibody, and the other is from a human Germline sequence. Framework regions of a known mature antibody typically comprise somatic cell mutation sites, possibly bringing about potential immunogenicity. Compared to a mature antibody, framework regions of a human Germline sequence theoretically have lower immunogenicity; moreover, the framework regions have a more flexible structure and strong plasticity, and are easy to accept different CDR regions. There is a bias with respect to the usage frequency of human antibody Germline genes in the human body. An antibody humanized by selecting and using a Germline framework region having a high usage frequency has advantages such as low immunogenicity, high expression level, and stable structure.

In a preferred embodiment of the present invention, a Germline sequence having the highest similarity to a murine antibody is not selected when performing humanization. Instead, similarity and usage frequency in the human body are both considered, and after extensive experimental screening, framework regions having preferred sequences are selected to perform humanization. Human antibody Germline framework regions are selected and used to perform CDR transplantation, and the humanized antibodies thus constructed have a more stable structure, a high expression level, a low immunogenicity, and a higher druggability.

In another preferred embodiment, the heavy chain constant region and/or light chain constant region may be a humanized heavy chain constant region or light chain constant region. More preferably, the humanized heavy chain constant region or light chain constant region is the heavy chain constant region of human IgG1, IgG2, etc., or the human kappa or lambda light chain constant region.

In another preferred embodiment, the sequence formed by adding, deleting, modifying, and/or substituting at least one amino acid sequence is preferably an amino acid sequence having least 80%, preferably at least 85%, more preferably at least 90%, and most preferably at least 95% homology.

The antibody of the present invention may be a double-stranded or single-stranded antibody, and may preferably be a fully humanized antibody.

The antibody derivatives according to the present invention may be single-chain antibodies, and/or antibody fragments, such as Fab, Fab', (Fab')2, or other known antibody derivatives in the art, as well as any one or more of IgA, IgD, IgE, IgG, and IgM antibodies or other subtypes thereof.

The antibody of the present invention may be a humanized antibody or CDR-grafted and/or modified antibody targeting CLDN18.2.

In the above content of the present invention, the number of the amino acids added, deleted, modified and/or substituted is preferably no more than 40%, more preferably no more than 35%, more preferably 1% to 33%, more preferably 5% to 30%, more preferably 10%to 25%, and more preferably 15% to 20% of the total number of amino acids in the initial amino acid sequence.

### Preparation of antibodies

Any method suitable for producing monoclonal antibodies may be used to produce the CLDN18.2 antibody of the present invention. For example, an animal may be immunized with a joined or naturally occurring CLDN18.2 protein or a fragment thereof. Suitable immunization methods may be used, including adjuvants, immunostimulants, and repeated booster immunization, and one or more means can be used.

Any suitable form of CLDN18.2 may serve as an immunogen (antigen) for generating a non-human antibody specific to CLDN18.2, and the antibody is screened for biological activities. The immunogen may be used alone or in combination with one or more immunogenic enhancers known in the art. The immunogen may be purified from a natural source, or produced in a genetically modified cell. The DNA encoding the immunogen may either be genomic or non-genomic (such as cDNA) in terms of source. A suitable genetic vector may be used to express the DNA encoding the immunogen. The vector includes but is not limited to adenovirus vectors, baculovirus vectors, plasmids, and non-viral vectors.

The humanized antibody may be selected from any type of immunoglobulin, including IgM, IgD, IgG, IgA, and IgE. Likewise, any type of light chain may be used in the compound and method herein. Specifically, the κ or λ chain or variant thereof may be used in the compound and method of the present invention.

The sequence of the DNA molecule for the antibody or fragment thereof of the present invention may be obtained using conventional techniques, such as PCR amplification or genomic library screening, among other methods. In addition, the coding sequences of the light and heavy chains may be fused together to form a single-chain antibody.

Once relevant sequences are obtained, a recombination method may be used to obtain the relevant sequences in large quantities. This typically involves cloning the relevant sequences into a vector, then transferring the vector into a cell, and then isolating the relevant sequences from the proliferated host cell by a conventional method so as to obtain the relevant sequences.

In addition, the relevant sequences may also be synthesized artificially, especially in the case that the fragments are short in length. In general, a fragment having a very long sequence may be obtained by first synthesizing a plurality of small fragments and then joining same. The DNA sequence may then be introduced into various existing DNA molecules (or such as vectors) and cells known in the art.

The term "nucleic acid molecule" refers to DNA molecules and RNA molecules. The nucleic acid molecule may be single-stranded or double-stranded, but preferably double-stranded DNA. When a nucleic acid is placed in a functional relationship with another nucleic acid sequence, the nucleic acid is "operably linked." For example, if a promoter or enhancer affects the transcription of a coding sequence, the promoter or enhancer is operably linked to the coding sequence.

The term "vector" refers to a nucleic acid molecule capable of transporting another nucleic acid to which it has been linked. In one embodiment, the vector is a "plasmid", which refers to a circular double-stranded DNA loop into which an additional DNA segment may be linked.

The present invention further relates to vectors comprising the above-mentioned suitable DNA sequence and a suitable promoter or control sequence. These vectors can be used to transform an appropriate host cell to enable the host cell to express the protein.

The term "host cell" refers to a cell into which an expression vector has been introduced. The host cell may be prokaryotic cells, such as bacterial cells; or lower eukaryotic cells, such as yeast cells; or higher eukaryotic cells, such as plant or animal cells (e.g., mammalian cells).

The step of transforming the host cell with the recombinant DNA according to the present invention may be performed using techniques well known in the art. The obtained transformant may be cultured using a conventional method, whereby the transformant expresses the polypeptide encoded by the gene of the present invention. Culture is carried out in a conventional medium under suitable conditions according to the host cell used.

Generally, the host cell obtained from the transformation is cultured under conditions suitable for the expression of the antibody of the present invention. Then, a conventional immunoglobulin purification step is performed by conventional isolation and purification means well known to a person skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of the monoclonal antibody may be determined using immunoprecipitation or an in vitro binding assay (e.g., radioimmunoassay (RIA) or enzyme-linked immunosorbent assay (ELISA)).

### Antibody formulations

An antibody has different stabilities in different formulation buffer solutions, manifested by a change in charge heterogeneity, degradation of the antibody molecule, polymerization, etc. These changes in quality properties are related to the physical and chemical properties of the antibody per se. Therefore, in the development of antibody drugs, it is necessary to screen for formulation buffer solutions that are suitable for different antibodies according to the physical and chemical properties of the antibodies. Currently, commonly used antibody formulation buffer systems include phosphate buffers, citric acid buffers, histidine buffers, etc. At the same time, different concentrations of salt ions or excipients such as sorbitol, trehalose, and sucrose as well as suitable amounts of surfactants such as Tween may be added according to the properties of the antibody so as to maintain the stability of the antibody.

### Pharmaceutical composition

The present invention further provides a composition. In a preferred embodiment, the composition is a pharmaceutical composition containing the aforementioned antibody or an active fragment, fusion protein or ADC thereof, or a corresponding CAR-T cell, and a pharmaceutically acceptable carrier. Generally, these substances may be formulated in a non-toxic, inert, and pharmaceutically acceptable aqueous carrier medium, in which the pH is typically about 5 to 8, and preferably about 6 to 8, although the pH value may vary depending on the properties of the substances formulated and the disease to be treated. The formulated pharmaceutical composition may be administered through conventional means, including (but not limited to) intratumoral, intraperitoneal, intravenous, or topical administration.

The antibody of the present invention may also be intracellularly expressed by a nucleotide sequence so as to be used for cell therapy, such as for chimeric antigen receptor T cell (CAR-T) immunotherapy, etc.

The pharmaceutical composition of the present invention may be directly used for binding to CLDN18.2 protein molecules, and thus may be used for the prevention and treatment of CLDN18.2 related diseases. In addition, other therapeutic agents may also be used at the same time.

The pharmaceutical composition of the present invention contains a safe and effective amount (such as 0.001wt% to 99wt%, preferably 0.01wt% to 90wt%, and more preferably 0.1wt% to 80wt%) of the monoclonal antibody (or a conjugate thereof) of the present invention as described above, and a pharmaceutically acceptable carrier or excipient. Such a carrier includes (but is not limited to): saline, buffer, glucose, water, glycerol, ethanol, and a combination thereof. The pharmaceutical formulation should match the mode of administration. The pharmaceutical composition of the present invention may be formulated in the form of an injection prepared, for example, by a conventional method using physiological saline or an aqueous solution containing glucose and other adjuvants. The pharmaceutical composition, such as an injection or a solution, is desirably manufactured under a sterile condition. The administration dosage of the active ingredient is a therapeutically effective amount, such as about 1 µg/kg body weight to about 5 mg/kg body weight per day. In addition, the polypeptide of the present invention may also be used in conjunction with other therapeutic agents.

When the pharmaceutical composition is used, a safe and effective amount of the pharmaceutical composition is administered to a mammal. The safe and effective amount is typically at least about 10 mg/kg body weight, and in most cases does not exceed about 50 mg/kg body weigh. Preferably, the dosage is about 10 mg/kg body weight to about 20 mg/kg body weight. Of course, for specific dosages, factors such as the route of administration and the patient's health status should also be considered, all of which are within the skill of an experienced physician.

### Detection use and kit

The antibody of the present invention may be used for detection application, such as for detection of a sample, so as to provide diagnostic information.

In the present invention, samples (sampled objects) used include cells, tissue samples, and biopsy specimens. The term "biopsy" used in the present invention should include all types of biopsies known to those skilled in the art. Therefore, the biopsies used in the present invention may include tissue samples prepared, for example, by endoscopic methods or by organ puncture or needle biopsy.

The samples used in the present invention include fixed or preserved cell or tissue samples.

The present invention further provides a kit containing the antibody (or a fragment thereof) of the present invention. In a preferred embodiment of the present invention, the kit further comprises a container, a user manual, a buffer, etc. In a preferred embodiment, the antibody of the present invention may be immobilized on a detection plate.

**Main advantages of the present invention include:**
1. The single domain antibody (VHH domain) against Claudin 18.2 of the present invention has high specificity and affinity.
2. The affinity of the anti-Claudin 18.2 antibody of the present invention to CLDN18.2 is at least 100 times higher than its affinity to CLDN18.1 (cell based).
3. The Claudin 18.2 CAR T cells of the present invention exhibit specific in vitro cytotoxicity towards Claudin 18.2 positive target cells, which is significantly higher than the in vitro killing efficiency towards Claudin 18.1 target cells.
4. The Claudin 18.2 CAR T cells of the present invention can effectively inhibit the growth of Claudin18.2 positive tumors in vivo and exhibit a long-lasting anti-tumor effect.
5. The Claudin 18.2 CAR05 T cells of the present invention exhibit stronger immune memory phenotypes during continuous culture in vitro.
6. The humanized Claudin 18.2 CAR T cells of the present invention show neither significant reduced cytotoxicity towards nor significantly decreased affinity to target cells compared to their maternal CAR-T cells.
7. The antibody of the present invention may be used for the specific detection of Claudin 18.2 protein and/or Claudin 18.1 protein.

The present invention is further described below in conjunction with specific examples. It should be understood that these examples are only used to illustrate the present invention and are not used to limit the scope of the present invention. Experimental methods without specific conditions indicated in the following examples usually follow conventional conditions, for example the conditions described in Sambrook et al., Molecular Cloning: A Laboratory Manual (New York: Cold Spring Harbor Laboratory Press, 1989), or according to conditions recommended by the manufacturers. Unless otherwise specified, percentages and parts are percentages by weight and parts by weight.

Unless otherwise specified, the materials and reagents used in the examples of the present invention are all commercially available products.

### Example 1

### 1. Screening for alpaca antibodies

A phage antibody library was screened for antibodies. The process for establishing an alpaca antibody library is briefly described as follows:

The antigen Claudin 18.2 VLP (0.5 mg) and 293-Claudin 18.2 cells (2e7) were injected to the neck of alpacas through multi-site injection. After multiple rounds of immunization, 50 ml of peripheral blood was collected for antibody titer detection and nanobody library construction.

### 1.1 Construction of a VHH phage display library

After multiple rounds of immunization of the alpacas, their serum titer satisfied: 1) ELISA titer > 32000, and 2) FACS titer > 200, and met the standards for library construction.

PBMCs were separated, and total RNA of the PBMCs was extracted and reverse transcribed into cDNA. VHH was amplified through nested PCR, and the VHH fragments were inserted into phagemids. The phagemids were amplified and harvested to obtain an antibody library.

The VHH3 antibody library was determined to have a library capacity of 5.2 × 10⁹ and a recombination efficiency (number of clonal PCR-positive clones/total number of clones) of 90%, meeting the relevant indexes of high-quality immune libraries.

### 1.2 Screening for Claudin18.2 antibody by cell panning

The process is briefly described as follows:
1.2.1 Pretreatment: Claudin 18.1 over-expressing cells (as negative cells) were used and added to the antibody library;
1.2.2 Binding step: step 1 was centrifuged and supernatant was obtained, the pretreated antibody library was used to bind to Claudin18.1 and Claudin18.2 over-expressing cells respectively, and screening was performed in liquid phase;
1.2.3 Rinsing: rinsing was performed 6 to 8 times;
1.2.4 Amplification: NEBalpha5F' cells were infected, helper phage was added, and culture was performed overnight;
1.2.5 Plating was performed to determine the degree of enrichment (the enrichment was considered successful if the number of positive cell-enriched clones/number of negative cell-enriched clones was greater than 10); and
1.2.6 The phages of R1 round were harvested and enriched for the next round of screening.

### 1.3 Identification of the clones obtained by cell panning - phage flow cytometry (phage-based FACS)

1.3.1 Identification of the clones obtained by cell panning: clones that bound to Claudin18.2 over-expressing cells were randomly selected, expanded culture was performed to obtain phages, and the binding affinity to Claudin 18.1 and Claudin 18.2 over-expressing cells was identified.

### 1.3.1 Cell preparation:

The Claudin18.1 and 18.2 over-expressing cells were centrifuged at 2×10⁵ cells/group, and washed with DPBS three times;

### 1.3.2 Sample incubation

The samples were respectively added with supernatant of the phages obtained from the expanded culture, and were incubated at 4°C for 2 h;

### 1.3.3 Incubation with secondary antibody

PE-labeled anti-M13 antibody (at a concentration of 5 ug/ml, 100 ul) was added, and incubation was performed at 4°C for 45 min;

### 1.3.4 Washing with DPBS

Washing with DPBS was performed three times, followed by resuspending in 300 ul of DPBS.

### 1.3.5 Flow cytometry detection

The resuspended cells were detected using a flow cytometer. The results of the fluorescence value of the cells showed that 14 random clones all bound to the Claudin 18.2 over-expressing cells, but did not bind to the Claudin18.1 over-expressing cells.

### 1.4 Sequencing of the positive clones

The 14 positive clones (from 1.3) were selected for sequencing. The clone numbers and VHH domain sequence IDs are shown in the table below. In the table, CDR1, CDR2, and CDR3 are defined based on the Kabat numbering. See Deschacht et al., 2010. Journal of Immunology, 184: 5696-704). Clones 7, 8 and 9 are identical to clones 12 and 13 in terms of CDRs 1, 2 and 3, the only difference being the FR regions, as shown in Table 1 below.

**Table 1**

| **Clone ID** | **VHH domain (SEQ ID NO)** | **CDR1** | **CDR2** | **CDR3** |
|---|---|---|---|---|
| 1 | SEQ ID NO: 1 | LMTTG (SEQ ID NO: 36) | TITFGGTTNYADSVKG (SEQ ID NO: 37) | DVIVGFQDRRVA (SEQ ID NO: 38) |
| 2 | SEQ ID NO: 2 | INYMG (SEQ ID NO: 39) | TITRGGSTNYQGNLKG (SEQ ID NO: 40) | RINFGILGGLRDY (SEQ ID NO: 41) |
| 3 | SEQ ID NO: 3 | NYNVA (SEQ ID NO: 42) | GATWSGRITNYSDSVRG (SEQ ID NO: 43) | NSFQLGDKPGDYH Y (SEQ ID NO: 44) |
| 4 | SEQ ID NO: 4 | LNYLA (SEQ ID NO: 45) | LVSSGGSTQYGDSVKG (SEQ ID NO: 46) | HTYDFLGRDY (SEQ ID NO: 47) |
| 5 | SEQ ID NO: 5 | SYIMG (SEQ ID NO: 48) | GITWSGGIKDYADSVKG (SEQ ID NO: 49) | NAIQLTRRSGDYAY (SEQ ID NO: 50) |
| 6 | SEQ ID NO: 6 | INYMG (SEQ ID NO: 39) | TITRGGSTNYQGNLKG (SEQ ID NO: 40) | RINFGILGGLRDY (SEQ ID NO: 41) |
| 7 | SEQ ID NO: 7 | LNFMG (SEQ ID NO: 51) | IATSGGSIQYGDSVKG (SEQ ID NO: 52) | HTYDFLGRDY (SEQ ID NO: 53) |
| 8 | SEQ ID NO: 8 | LNFMG (SEQ ID NO: 51) | IATSGGSIQYGDSVKG (SEQ ID NO: 52) | HTYDFLGRDY (SEQ ID NO: 53) |
| 9 | SEQ ID NO: 9 | LNFMG (SEQ ID NO: 51) | IATSGGSIQYGDSVKG (SEQ ID NO: 52) | HTYDFLGRDY (SEQ ID NO: 53) |
| 10 | SEQ ID NO: 10 | INYMG (SEQ ID NO: 39) | TITRGGTTNYQGDLKG (SEQ ID NO: 54) | RINFGILGGLRDY (SEQ ID NO: 41) |
| 11 | SEQ ID NO: 11 | INYMG (SEQ ID NO: 39) | IITRGGSTTYAPSLKG (SEQ ID NO: 55) | RINFGILGGLRDY (SEQ ID NO: 41) |
| 12 | SEQ ID NO: 12 | LNFMG (SEQ ID NO: 51) | IATSGGSIQYGDSVKG (SEQ ID NO: 52) | HTYDFLGRDY (SEQ ID NO: 53) |
| 13 | SEQ ID NO: 13 | LNFMG (SEQ ID NO: 51) | IATSGGSIQYGDSVKG (SEQ ID NO: 52) | HTYDFLGRDY (SEQ ID NO: 53) |
| 14 | SEQ ID NO: 14 | IITMG (SEQ ID NO: 56) | TVTRGGRLNYGDSVKG (SEQ ID NO: 57) | ILESGNF (SEQ ID NO: 58) |

### 1.5 NGS sequencing of phages obtained by cell panning and enrichment

The phage pool obtained by panning and enrichment using 293FT-Claudin 18.2 over-expressing cells was subjected to PCR to amplify VHH3 gene fragments, and NGS sequencing was performed to obtain thirty VHH3 gene sequences.

The thirty VHH3 genes were further tested for the performance of binding of the expressed VHH domains to Claudin 18.2, and six nanobodies having optimal performance were obtained. The sequences of the VHH domains and the sequences of CDR1, CDR2, and CDR3 of these nanobodies are shown in Table 2.

**Table 2: NGS sequencing analysis of phages obtained by cell panning and enrichment**

| Clone ID | VHH domain (SEQ ID NO) | CDR1 | CDR2 | CDR3 |
|---|---|---|---|---|
| 15 | SEQ ID NO: 15 | TSPSAMG (SEQ ID NO: 59) | TIGWTGGKQYYADSVKG (SEQ ID NO: 60) | RRAGYTGTPTRGDSYEH (SEQ ID NO: 61) |
| 16 | SEQ ID NO: 16 | YWAIS (SEQ ID NO: 62) | CIRFSDGSTYDADSVKG (SEQ ID NO: 63) | DMWADCGSMEYDY (SEQ ID NO: 64) |
| 17 | SEQ ID NO: 17 | LMTTG (SEQ ID NO: 36) | TITFGGTTNYADSVKG (SEQ ID NO: 37) | DVIVGFQDRRVA (SEQ ID NO:38) |
| 18 | SEQ ID NO: 18 | LMTTG (SEQ ID NO: 36) | TITFGGTTNYADAVKG (SEQ ID NO: 65) | DYIFGFGDRRVA (SEQ ID NO: 66) |
| 19 | SEQ ID NO: 19 | INYAG (SEQ ID NO: 67) | LITRGGSTTYGASLKG (SEQ ID NO: 68) | RINFGILGGLRDY (SEQ ID NO: 41) |
| 20 | SEQ ID NO: 20 | INYMG (SEQ ID NO: 39) | LITRGGTTNYAESLKG (SEQ ID NO: 69) | RINFGILGGLRDY (SEQ ID NO: 41) |

### 1.6 Determination of the affinity of the preferred sequences - ELISA

The affinity of the recombinant antibodies were determined based on ELISA method. Four sequences (clones 1, 5, 8, and 10) were selected from the 14 clones to synthesize VHH-hIgG1Fc recombinant antibodies, and the affinity of the antibodies to antigens was determined. The antigens were Claudin 18.2 VLP and Claudin18.2 recombinant protein (His tag), respectively. The ELISA EC50 data are shown in Table 2.

The determination method was as follows:
1.5.1 Coating: on day 1, the antigens (200 ng/well) were coated onto an ELISA plate at 4°C overnight;
1.5.2 Blocking: on day 2, the ELISA plate was blocked with 0.5% BSA at room temperature for 2 h;
1.5.3 Binding: on day 2, the VHH hIgG1 Fc recombinant antibodies were diluted at 1:3 starting from 10 µg/mL to obtain seven concentrations, and the dilutions were added to the ELISA plate at 100 µL/well at room temperature for 2 h;
1.5.4 Washing: the ELISA plate was rinsed with PBS-Tween, then added with 100 µL of anti-human IgG1Fc HRP antibody, and placed at room temperature for 1 h;
1.5.5 Color development: the ELISA plate was rinsed with PBS-Tween, then rinsed with PBS, added with 100 µL of TMB, and placed at 37°C for 5 to 10 min;
1.5.6 Termination: 50 µL of 1 M phosphoric acid was added to terminate the reaction; and
1.5.7 Measurement: the absorbance value at 450 nm was measured using an ELISA reader, the results being shown in Table 3.

**Table 3**

| | Claudin18.2 VLP (200 ng/well) | | | | Claudin 18.2-his (200 ng/well) | | |
|---|---|---|---|---|---|---|---|
| Concentration (ug/mL) | 1-hIgG1Fc | 5-hIgG1Fc | 8-hIgG1Fc | 10-hIgG1Fc | 1-hIgG1Fc | 5-hIgG1Fc | 10-hIgG1Fc |
| 100 | 2.657 | 2.353 | 2.464 | 2.623 | 2.361 | Overflow | 3.379 |
| 3.333 | 2.126 | 2.138 | 2.103 | 2.512 | 1.083 | 3.499 | 2.149 |
| 1.111 | 1.355 | 1.859 | 1.872 | 2.141 | 0.473 | 2.530 | 1.065 |
| 0.37 | 0.585 | 0.942 | 0.931 | 1.458 | 0.216 | 1.411 | 0.476 |
| 0.123 | 0.293 | 0.39 | 0.474 | 0.568 | 0.100 | 0.463 | 0.172 |
| 0.041 | 0.271 | 0.275 | 0.337 | 0.355 | 0.070 | 0.207 | 0.084 |
| 0.014 | 0.282 | 0.274 | 0.326 | 0.329 | 0.066 | 0.108 | 0.063 |
| 0 | 0.275 | 0.247 | 0.289 | 0.311 | 0.060 | 0.057 | 0.054 |
| EC50 (ug/mL) | 1.46 | 0.55 | 0.61 | 0.40 | 15.68 | 0.81 | 4.00 |
| EC50 (nM) | 18.28 | 6.87 | 7.66 | 4.96 | 196 | 10.1 | 50 |

As shown in Table 3, the recombinant antibodies of the present disclosure all bound to Claudin18.2 VLP or Claudin18.2-his antigen in a dose-dependent manner, and their binding efficacy was in the range of from 4 nM to 200 nM (EC50 value).

### 1.7 Determination of the affinity of the preferred sequences - FACS

The ability of the recombinant antibodies to bind to 293FT-hClaudin18.1 cells (negative cells) and 293FT-hClaudin18.2 cells (positive cells) was determined using cell-based flow cytometry. The 293FT-Claudin 18.1 cells and 293FT-Claudin18.2 cells were incubated with recombinant antibodies of the four preferred sequences (5 ug/mL, and 10 ug/mL) at 4 °C for 30 min, respectively, and were then washed with DPBS. After washing, the cell precipitate was resuspended in secondary antibody (1:100, Goat anti human IgG (H+L), FITC), then incubated in dark for 15 min, and then washed with DPBS. The cells were subjected to flow cytometry to determine the level of binding of the recombinant antibodies to 293FT-hClaudin18.1 cells and 293FT-hClaudin18.2 cells. The recombinant antibodies were each labeled with an Fc tag and were designated as 1-hlgG1Fc, 5-hlgG1Fc, 8-hlgG1 Fc, and 10-hlgG1Fc, respectively. The recombinant antibodies were used at a concentration of 10 ug/mL. The binding ability of each of the recombinant antibodies determined was denoted as binding percentage, which was calculated as (cells with antibody binding/total cells) x 100%.

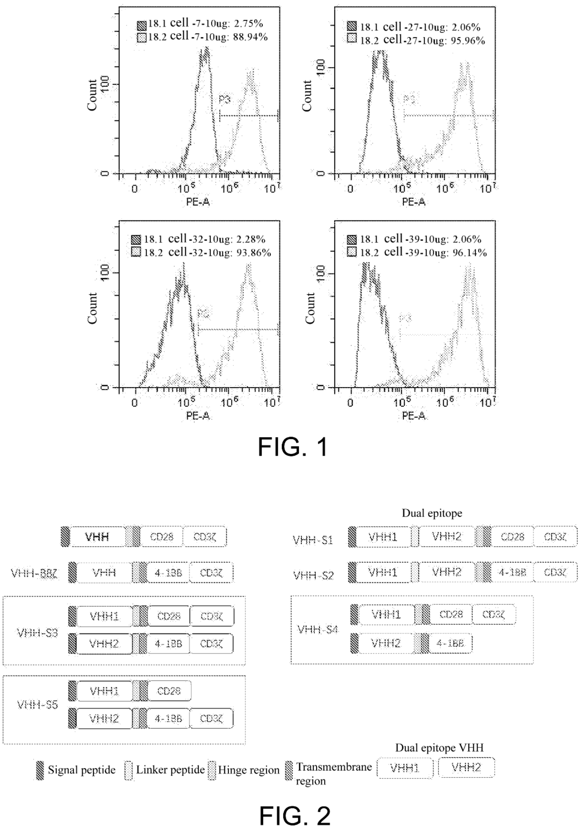

The results are shown in Figure 1. The binding percentage of the recombinant antibodies to Claudin 18.1 cells were 2.75%, 2.06%, 2.26%, and 2.06%, respectively, and the binding percentage thereof to Claudin18.2 cells were 88.94%, 95.96%, 93.86%, and 96.1%, respectively, indicating the binding specificity of the recombinant antibodies to human Claudin18.2.

### 1.8 Humanized anti-Claudin 18.2 antibodies

Humanized anti-Claudin 18.2 antibodies were obtained based on the Claudin18.2 VHH antibodies, which were subjected to humanization reconstruction according to references such as Vincke et al., Journal of Biochemistry, 284(5):3273-3284(2009). The framework regions of the humanized VHH domains disclosed in the present application were reconstructed on that basis to reduce antigenicity. In the design of humanized single-domain antibodies from Camelidae species, the numbering of the characteristic residues in their VHH was based on Kabat, such as residues 11, 37, 44, 45, and 47 (Muyldermans, Molecular Biotechnology Review, 74:277-302(2001). The humanized sequences are shown in Table 4.

**Table 4**

| Clone ID | VHH domain (SEQ ID NO.) | CDR1 | CDR2 | CDR3 |
|---|---|---|---|---|
| 5H1 | SEQ ID NO: 21 | SYIMG (SEQ ID NO: 48) | GITWSGGIKDYADSVKG (SEQ ID NO: 49) | NAIQLTRRSGDYAY (SEQ ID NO: 50) |
| 5H2 | SEQ ID NO: 22 | | | |
| 5H3 | SEQ ID NO: 23 | | | |
| 5H4 | SEQ ID NO: 24 | | | |
| 18H1 | SEQ ID NO: 25 | LMTTG (SEQ ID No: 36) | TITFGGTTNYADAVKG (SEQ ID NO: 65) | DYIFGFGDRRVA (SEQ ID NO: 66) |
| 2H1 | SEQ ID NO: 26 | INYMG (SEQ ID NO: 39) | TITRGGSTNYQGNLKG (SEQ ID NO: 40) | RINFGILGGLRDY (SEQ ID NO: 41) |

### 1.9 Exemplary designs of single CAR and dual epitope CAR of chimeric antigen receptor targeting Claudin 18.2

Exemplary structures of Claudin 18.2 single CAR and dual CAR were designed, as shown in Figure 2. Exemplary amino acid sequences of the CAR constructs are listed in Table 5.

### Example 2 Preparation of CAR-T targeting Claudin18.2

### 2.1 Preparation of CAR-T targeting Claudin18.2

### General materials and methods:

Peripheral blood mononuclear cells (PBMCs) were separated from donor blood and T cells were expanded. The peripheral blood mononuclear cells (PBMCs) were isolated by a density gradient centrifuge using Ficoll. Then, T cells were enriched, activated by magnetic beads coupled with anti-CD3/anti-CD28, cultured, and expanded.

### Target cell lines, PBMCs, and CAR-T culture:

1. 293FT-Luc, 293FT-CLDN18.1-luc, 293FT-CLN18.2-Luc cell lines, AGS-CLDN18.2-Luc, AGS-CLDN18.1(KO CLDN18.2)-Luc (KO CLDN18.2 refers to knocking out endogenous Claudin18.2), Aspc-1-CLDN 18.2-Luc, and 293FT series cells were cultured in DMEM medium. The cells labeled with the Luc tag all stably expressed firefly luciferase. AGS-CLDN 18.2-Luc and AGS-CLDN 18.1 (KO CLDN18.2)-Luc series cells were cultured in F12 medium, and Aspc-1-CLDN 18.2-Luc cells were cultured in RPMI1640 medium. The RPMI1640, F12, and DMEM media were all supplemented with 10% (v/v) fetal bovine serum, 100 U/ml penicillin and streptomycin, 2 mM glutamine, and 1 mM sodium pyruvate. All of the cells were cultured in a 37°C, 5% CO2 incubator.
2. CAR-T cells were cultured in X-vivo15 medium (containing 5% FBS, 2 mM L-glutamine, 1 mM sodium pyruvate, and 300 IU/ml rhIL2). The CAR-T cell culture medium was added with rhIL-2. Every two days, 300 IU/ml of IL-2 was added. All of the cells were cultured at 37°C under 5% CO2.

### CAR-T preparation

The VHH coding sequences of the selected antibodies were optimized by using Gene codon optimization. The nucleotide molecules encoding chimeric antigen receptor (CAR) skeleton polypeptides were synthesized, and cloned into a lentiviral vector (pCDH skeleton vector carrying a GFP expression sequence). The flag tag was inserted into the N-terminus of the hinge region. The VHH coding sequences were selected from Tables 1, 2, and 4 and corresponded to their CAR-T numbering, as specifically shown in Table 5. The control antibody (IMAB) had an IMAB362 sequence, including heavy and light chain variable region fragments thereof.

A mixture containing a lentivirus packaging plasmid and a vector expressing a CAR construct were pre-mixed with polyetherimide (PEI) in a pre-optimized ratio, and incubated at room temperature for 15 to 20 min. Then the transfection mixture was added to HEK293 cells, and the cells were cultured for 48 to 72 h. The supernatant was collected, and the lentivirus was concentrated.

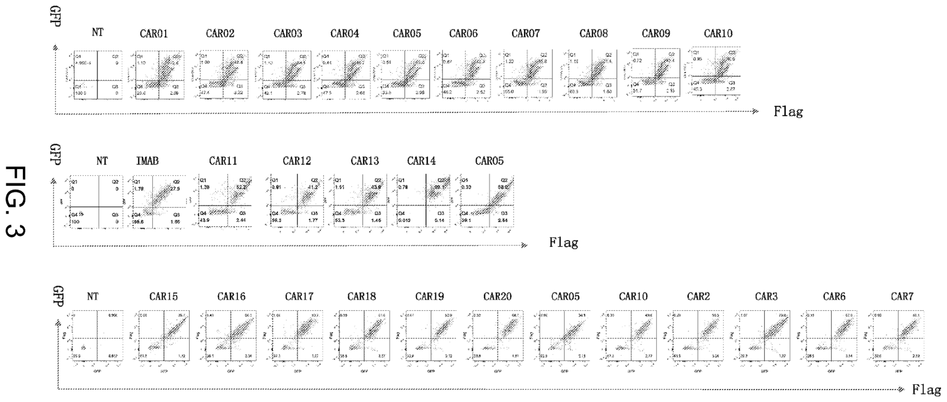

Primary T cells activated by Dynabeads CD3/CD28 were transfected with the lentivirus. Three days after transfection, the percentage of positive cells expressing CAR was determined using an anti-flag tag antibody, and at the same time, the FITC channel was determined (showing GFP expression). Three batches of CAR-T cells were produced separately, and their CAR positive rate was determined to range between 30% and 70%. Flag and GFP were showed to be expressed in a ratio of close to 1:1, indicating successful co-expression of CAR and GFP, as seen in Figure 3.

**Table 5 Table showing the correspondence of CAR VHH amino acid sequences and corresponding VHH domains for preparing CAR-T cells targeting Claudin18.2**

| CAR number | SEQ ID NO.: | CAR number | SEQ ID NO.: |
|---|---|---|---|
| CAR01 | SEQ ID NO.1 | CAR14 | SEQ ID NO.14 |
| CAR02 | SEQ ID NO.2 | CAR15 | SEQ ID NO.15 |
| CAR03 | SEQ ID NO.3 | CAR16 | SEQ ID NO.16 |
| CAR04 | SEQ ID NO.4 | CAR17 | SEQ ID NO.17 |
| CAR05 | SEQ ID NO.5 | CAR18 | SEQ ID NO.18 |
| CAR06 | SEQ ID NO.6 | CAR19 | SEQ ID NO.19 |
| CAR07 | SEQ ID NO.7 | CAR20 | SEQ ID NO.20 |
| CAR08 | SEQ ID NO.8 | CAR21 | SEQ ID NO.21 |
| CAR09 | SEQ ID NO.9 | CAR22 | SEQ ID NO.22 |
| CAR10 | SEQ ID NO.10 | CAR23 | SEQ ID NO.23 |
| CAR11 | SEQ ID NO.11 | CAR24 | SEQ ID NO.24 |
| CAR12 | SEQ ID NO.12 | CAR25 | SEQ ID NO.25 |
| CAR13 | SEQ ID NO.13 | CAR26 | SEQ ID NO.26 |

### Example 3 In vitro function of CAR-T cells targeting Claudin18.2

### 3.1 Analysis of CAR-T cell memory phenotypes

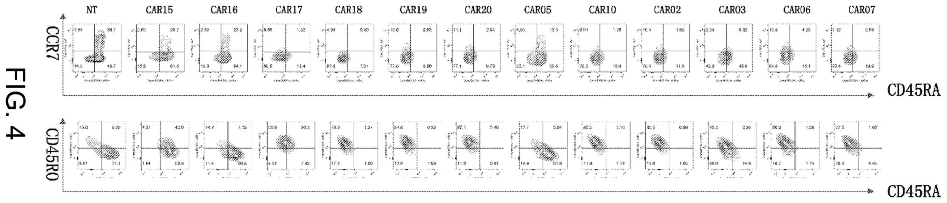

After culturing CAR-T continuously for 8 to 10 days, the cell phenotype was determined using a flow cytometer. An anti-flag antibody was used to distinguish CAR positive and negative cells. At the same time, CD45RA, CD45RO, and CCR7 antibodies were used to determine T cell memory phenotypes. Gating on CAR positive cells, the Naïve, T_{CM}, T_{EM}, and T_{EMRA} cell populations were analyzed. As shown in Figure 4. The CD45RA⁺ to CCR7⁺ (Naive cell) ratio for CAR15, CAR16, and CAR05 was higher than that of the other groups (20%, 23%, and 13% vs about 2%), and the CD45RA⁺ to CD45RO⁻ cell ratio was also higher than that of the other groups, indicating a higher level of youngness of the cells compared to the other CAR-T cells.

### 3.2 Cytotoxicity of CAR-T cells targeting Claudin18.2

Target cells expressing luciferase were constructed, including 293FT-Luc, 293FT-Claudin18.1-Luc, and 293FT-Claudin18.2-Luc. When performing determination, after addition of luciferin as a substrate, luciferase can react with luciferin to produce fluorescence. The number of live cells among the target cells can be determined by measuring the activity of luciferase by determining the intensity of the fluorescence, thereby evaluating the cytotoxicity of CAR-T cells. Specific cytotoxicity is calculated by the following formula: specific cytotoxicity%=100% x (RLUonly - RLUsample/RLUonly). RLUsample refers to the activity of luciferase measured in the well containing CAR T cells targeting Claudin18.2, and RLU_{only} refers to the activity of luciferase measured in the well of the target cell-only group.

The luciferase-expressing target cells 293FT-Luc, 293FT-Claudin18.1-Luc, and 293FT-Claudin18.2-Luc cells were respectively incubated with CAR-T for 6 h at an effector to target ratio of (E: T=1:1) to compare cytotoxicity.

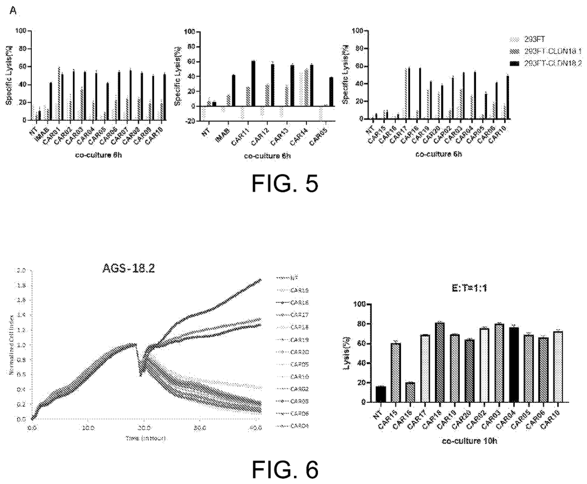

The results are shown in Figure 5. The three data bars from left to right represent the rates of lysis, by the CAR T cells, of the target cells 293FT-Luc, 293FT-Claudin18.1-Luc, and 293FT-Claudin18.2-Luc, respectively.

The results showed that IMAB, CAR02, CAR04 to CAR13, and CAR18 T cells showed no significant cytotoxicity to 293FT-Luc cells and 293FT-Luc cells that stably expressed human Claudin18.1. CAR01 and CAR17 CAR-T cells showed significant cytotoxicity to 293FT-Luc cells that stably expressed human Claudin18.1 and Claudin18.2, while CAR14 T cells showed significant cytotoxicity to 293FT-Luc, 293FT-Claudin18.1-Luc, and 293FT-Claudin18.2-Luc cells. In addition, CAR02, CAR05, and CAR18 T cells showed better killing specificity (the ratio of their lysis rate for 293FT-Claudin18.2-Luc cells/their lysis rate for 293FT-Claudin18.1-Luc cells was the highest).

The results of real-time cell analysis (RTCA) are shown in Figure 6. In the effector to target ratio of (1:1), CAR16 showed no significant cytotoxicity to AGS cells that stably expressed human Claudin18.2, and CAR15 showed lower cytotoxicity to AGS cells that stably expressed human Claudin18.2 compared to other CAR-Ts; except for CAR15 and 16, the CAR-Ts all showed significant cytotoxicity when co-incubated with the target cells AGS-Claudin18.2 for 10 h.

### 3.3 Release of cytokines

CAR-T cells and AGS-Claudin18.2 cells were mixed at 100 ul each, at a ratio of 1:1, in RPMI medium. The density was 1×10⁵/ml for each CAR-T cell. The cells were cultured overnight in a 96-well plate. Then the culture medium was collected and centrifuged. The supernatant was taken, and cytokines IFN-γ, IL-2, IL-4, IL-6, IL-10, and TNF released into the cell culture medium were determined using a cytokine detection kit (CBA kit, BD).

The detection results are shown in Figure 7. Large amounts of IL-2, TNFα and IFNγ were detected in the co-cultured supernatants of CAR17-CAR20, CAR02 to CAR06, and CAR10, indicating the specific release of cytokines including IL-2, TNFα and IFNγ during the action of the Claudin18.2 CAR-T cells on the CLDN18.2 positive target cells.

Advantageously, these CAR T cells of the present invention do not cause a significant release of IL-6, suggesting a low risk of inducing cytokine storms.

In addition, CAR05 T cells unexpectedly caused a high release of IL-2, TNFα and IFNγ (significantly higher than other CAR T cells).

### Example 4 In vitro data of humanized VHH domain CAR-T cells

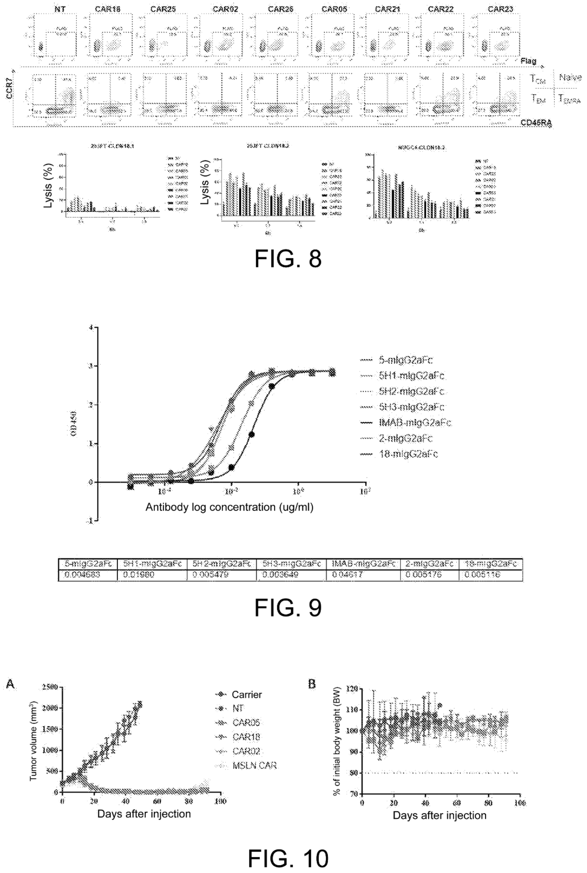

Using the same method for determining the in vitro function of CAR-T cells targeting Claudin18.2 in Example 3, the in vitro function of humanized VHH domain CAR-T cells was determined, as shown in Figure 8.

The CAR-T phenotypes after 8 days of culture were analyzed. The results showed besides a significant difference in phenotype between CAR21 and CAR05, the CD45RA+CCR7+(Naïve) cell ratio for CAR21 was significantly lower than that for CAR05, CAR22, and CAR23 (27.5% vs. 2.4%), and the T_{EMRA} cell ratio for CAR21 was significantly higher than that for CAR05 (61.4% vs. 43.9%). This was consistent with the instant killing result of CAR21, the instant killing rate being greater than CAR05.

The results are shown in Figure 8. The humanized domain CAR-T cells were compared with the maternal CAR-T cells in terms of instant killing efficacy. Among the humanized domain CAR-T cells, CAR21 (humanized) showed a higher instant killing efficacy against 293FT-CLDN18.2-Luc (80% vs. 50%) and Nugc4-CLDN18.2-Luc (70% vs. 45%) compared to CAR05 (non-humanized), while CAR22, CAR23 (humanized), and CAR05 (non-humanized) showed similar instant killing efficacies. CAR25 (humanized) and CAR18 (non-humanized) showed an instant lysis rate of 75% vs. 60% against 293FT-CLDN18.2-Luc, while CAR26 (humanized) and CAR02 (non-humanized) showed an instant lysis rate of 70% vs. 60% against 293FT-CLDN18.2-Luc. The results indicated that humanized CAR25 and CAR26 exhibited a higher instant killing efficiency than the maternal CAR-T.

### Example 5 Determination of affinity

### 5.1 Using ELISA to determine the binding curves of recombinant antibodies to Claudin18.2 VLP antigen

The recombinant antibodies in the table below were synthesized respectively, and ELISA was used to determine the binding of Claudin18.2-VLP protein to the aforementioned recombinant antibodies. The specific procedure of the ELISA experiment is as follows:

200 ng/well of Claudin18.2-VLP protein was added to an ELISA plate and the plate was coated overnight at 4°C. The plate was washed with PBS three times, added with 1% BSA/PBS at 200 uL/well, and blocked at 37°C for 1 h. After washing the plate with 100 ul PBS, gradient dilutions of the aforementioned fusion antibodies were added, and binding was allowed to proceed at 37°C for 1 h. The plate was rinsed with PBST three times. 100 ul of HRP-goat anti-mouse IgG Fc diluted at 1:5000 was added, and binding was allowed to proceed at 37°C for 1 h. The plate was rinsed with PBST three times. A TMB developing solution was added at 100 uL/well, and color development was allowed to proceed at 37°C for 10 min. 50 µL of 1 M phosphate was added to terminate the reaction, and the absorbance value at 450 nm was measured using an ELISA reader.

The results of the experiment to test the antigen binding ability of VHH using ELISA technology are shown in Figure 9. The EC₅₀ values of the tested nanobodies of the present invention were all superior to that of the positive control antibody IMAB.

On the basis the EC₅₀ values, the affinity abilities were ranked, from high to low, as 5H3>5>2,18, 5H2>5H1>IMAB.

### 5.2 Using flow cytometry to determine the binding ability of recombinant antibodies to 293FT-CLDN18.1-Luc or 293FT-CLDN18.2-Luc cells

293FT-Claudin 18.1 cells as negative cells and 293FT-Claudin18.2 cells as positive cells were respectively co-incubated with gradient dilutions of the fusion antibody anti-Claudin18.2 VHH-mlgG2aFc at 4°C for 1 h. The cells were rinsed with PBS three times, a secondary antibody (1:200 APC goat anti-mouse IgG Fc) was added, and incubation was allowed to proceed at 4°C for 30 min. The cells were rinsed with PBS once, and were subjected to flow cytometry. The results of determination of the VHH affinity using FACS technology are shown in Table 6.

**Table 6**

| Antibody ID | EC50, nM | |
|---|---|---|
| | Binding to 293FT CLDN18.1-Luc | Binding to 293FT CLDN18.2-Luc |
| 2-mIgG2aFc | 133.2nM | 0.02nM |
| 5-mIgG2aFc | N.D. | 0.013nM |
| 18-mIgG2aFc | N.D. | 0.06nM |
| IMAB-mIgG2aFc | N.D. | 0.07nM |

### 5.3 Using SPR technology to determine the affinity of recombinant antibodies to human Claudin18.2 protein

Six antibodies were diluted with a working reagent to 5 µg/mL. The dilutions were injected at a flow rate of 10 µL/min into the Protein A capture experimental channels (Fc2) for about 200 RU. Ligand capture did not need to be performed at the reference channel (Fc1).

Human Claudin-18.2 protein was diluted 2-fold with the working reagent (Table 5). The diluted human Claudin-18.2 protein was injected into the experimental channels (Fc2) and the reference channel (Fc1) successively at a flow rate of 30 µL/min, and binding and dissociation were allowed to proceed for corresponding times. The binding and dissociation steps were both conducted in the working reagent. After the analysis conducted for each concentration, it was necessary to regenerate the chip by using glycine hydrochloride having a pH value of 1.5 at a flow rate of 20 µL/min for 30 s to wash off the ligand and the undissociated analytes. When conducting analysis for the next concentration, the experimental channels (Fc2) needed to recapture the same amount of the ligand.

The KD value for each sample was calculated using the Biocore 8K analysis software Biacore Insight Evaluation Software. The reference channel (Fc1) was used for background subtraction.

The lower the dissociation constant KD value (the ratio of dissociation rate Kd/binding rate Ka), the higher the affinity of the antibody. The KD value varies depending on different complexes of antibodies and antigens, and depends on Ka and Kd.

The results are shown in Table 7. The KD values for H1-mlgG Fc, IMAB-mIgG Fc, 5H2-mlgG2a Fc, 5-mlgG2a Fc, and 2-mlgG2a Fc were 7.21 nM, 5.65 nM, 3.32 nM, 3.02 nM, and 1.1 nM, respectively. The ranking of the affinity of the antibodies (from high to low): 2-mlgG2a Fc, 5-mlgG2a Fc, 5H2-mlgG2a Fc, IMAB-mIgG Fc, and 5H1-mlgG Fc.

The ranking of Ka (from high to low): 2-mlgG2a Fc, IMAB-mlgG Fc, 5H2-mlgG2a Fc, 5-mlgG2a Fc, and 5H1-mlgG Fc.

The ranking of Kd (from high to low): IMAB-mlgG Fc, 5H1-mlgG Fc, 2-mlgG2a Fc, 5H2-mlgG2a Fc, and 5-mlgG2a Fc.

**Table 7 Results of determination of the affinity of four antibodies to human Claudin-18.2 protein**

| **Antibody No.** | **Ka (1/Ms)** | **Kd (1/s)** | **KD (nM)** |
|---|---|---|---|
| 5-mIgG Fc | 1.23E+05 | 3.73E-04 | 3.02nM |
| 5H1-mIgG Fc | 7.66E+04 | 5.52E-04 | 7.21nM |
| 5H2-mIgG Fc | 1.26E+05 | 4.17E-04 | 3.32nM |
| 2-mIgG Fc | 4.71E+05 | 5.20E-04 | 1.10nM |
| IMAB-mIgG Fc (Positive control) | 1.33E+05 | 7.53E-04 | 5.65nM |

### 5.4 Study on in vivo anti-tumor efficacy

The in vivo anti-tumor efficacy of Claudin18.2 CAR-T cells was evaluated in Aspc-1 18.2-Luc pancreatic cancer NOG-DKO mouse (NOD.Cg-B2mem1Tac Prkdcscid H2-Ab1tm1Doi II2rgtm1Sug/JicCrl) model.

2E6 AsPC-1 CLDN18.2-Luc cells (CLDN18.2+MSLN+) were subcutaneously injected. When the tumor load reached around 200 mm3, the mice were divided into groups of 3 to 4 mice per group. One day after grouping, 200 uL of CLDN18.2 CAR-T cells, MSLN CAR-T or NT cells were injected via the tail vein, 5E6 CAR-T/mouse. On Day 1 after CAR-T injection, small amounts of mouse blood were taken to determine the number of surviving CAR-T cells in vivo. Thereafter, blood samples were taken once a week to determine various phenotypes of the CAR-T cells, and subcutaneous tumor size and mouse weight were measured twice a week.

The results are shown in Figure 10. Claudin18.2 CAR-T cells and MSLN CAR-T cells showed a significant anti-tumor effect on transplanted tumor cells in vivo. VHH candidate CAR-T cells, including CAR02, 05, and 18, showed similar anti-tumor effects. By Day 80 and D90 after reinjection of the CAR-T cells, the mice still survived without tumor recurrence. When the control MSLN CAR-T against the MSLN target was used, although the tumor cells could also be killed, the tumor began to recur on Day 80 after reinjection of the CAR-T cells, and by Day 90, the tumor began to recur and the tumor volume reached about 200 mm³.

The in vivo experimental results showed that CAR-T (CAR02, 05, 18) cells targeting Claudin18.2 had strong anti-tumor activity and persistence.

**Table 8**

| **SEQ ID NO.** | **VHH domain sequence** |
|---|---|
| 1 | EVQLVGSGGGLVQPGGSLRLSCAASGSILNLMTTGWYR QAPGKQRDLVATITFGGTTNYADSVKGRFTISRDKSKNT AYLQMDNLKPEDTAVYFCYADVIVGFQDRRVAWDAGTQ VTVSS |
| 2 | EVQLVESGGGLVQPGGSLRLSCAASGNILSINYMGWYR QAPGKERELVATITRGGSTNYQGNLKGRFIISGDNTKDT VYLQMNNLQPEDTAVYYCYFRINFGILGGLRDYWGQGT QVTVSS |
| 3 | QVQLVESGGGLVQAGDSVRLSCAASGQTLSNYNVAWF RQAPGKEREFVAGATWSGRITNYSDSVRGRFTISRDNA |
| | KNTVYLQMNSLGPGDTAVYHCAANSFQLGDKPGDYHY WGQGTQVTVSS |
| 4 | EVQLVESGGGLVQPGGSLRLSCAASGQFFSLNYLAWYR QAPGSQRELIALVSSGGSTQYGDSVKGRFTISRDNAKNT VYLQMASLKPEDTAVYYCNLHTYDFLGRDYWGQGTQVT ISS |
| 5 | QVQLVESGGGLVQTGGSLRLSCAASDSTFSSYIMGWFR QAPGKEREFVAGITWSGGIKDYADSVKGRFTIARDNAKN TVYLQMNSLKPEDAAIYYCAANAIQLTRRSGDYAYWGQ GTQVTVSS |
| 6 | EVQLVESGGGLVQAGGSLRLSCAASGNILSINYMGWYR QAPGKERELVATITRGGSTNYQGNLKGRFIISGDNTKDT VYLQMNNLQPEDTAVYYCYFRINFGILGGLRDYWGPGT QVTVSS |
| 7 | EVQLVESGGGLVQPGGSLRLSCAASGSIFGLNFMGWW RQAPGSQRELVAIATSGGSIQYGDSVKGRFTISRDNAKN TVYLQMASLKPEDTAVYYCNVHTYDFLGRDYWGQGTQ VTVSL |
| 8 | QVQLVESGGGLVQPGGSLRLSCAASGSIFGLNFMGWW RQAPGSQRELVAIATSGGSIQYGDSVKGRFTISRDNAKN TVYLQMASLKPEDTAVYYCNVHTYDFLGRDYWGQGTQ VTVSL |
| 9 | EVQLVESGGGLVQPGGSLRLSCAASGSIFGLNFMGWW RQAPGSQRELVAIATSGGSIQYGDSVKGRFTISRDNAKN TVYLQMANLKPEDTAVYYCNVHTYDFLGRDYWGQGTQ VTVSL |
| 10 | QVQLAESGGGLVQPGGSLRLSCAASGNILSINYMGWYR QAPGKERELVATITRGGTTNYQGDLKGRFIISGDNTKDT VYLQMNNLQPEDTAVYYCYFRINFGILGGLRDYWGQGT QVTVSS |
| 11 | QVQLVESGGGLVQPGGSLRLSCAASGNILSINYMGWYR QAPGKERELVAIITRGGSTTYAPSLKGRFTISGDNAKDTV YLQMNSLQPEDTAVYYCNFRINFGILGGLRDYWGQGTQ VTVSS |
| 12 | QVQLVESGGGLVQAGGSLRLSCAASGSIFGLNFMGWW RQAPGSQRELVAIATSGGSIQYGDSVKGRFTISRDNAKN TVYLQMASLKPEDTAVYYCNVHTYDFLGRDYWGQGTQ VTVSL |
| 13 | QVQLVESGGGLAQLGGSLRLSCAASGSIFGLNFMGWW RQAPGSQRELVAIATSGGSIQYGDSVKGRFTISRDNAKN TVYLQMASLKPEDTAVYYCNVHTYDFLGRDYWGQGTQ VTVSL |
| 14 | EVQLVESGGGLVQPGGSLRLSCAASGSLFSIITMGWYR QAPGKQRELVATVTRGGRLNYGDSVKGRFTISSGNVEN TLDLQMNNLKPEDTAVYYCNAILESGNFWGRGTQVTVS S |
| 15 | QVQLVESGGGLVQAGGSLRLSCAASGLTFSTSPSAMG WFRQAPGKEREFVATIGWTGGKQYYADSVKGRFTISRD NVKNTGYLQMNSLKPEDTAVYYCAARRAGYTGTPTRGD SYEHWGQGTQVTVSS |
| 16 | QVQLVESGGGLVQPGGSLRLSCAASGFTLDYWAISWFR QAPGKEREGVSCIRFSDGSTYDADSVKGRFTISRDNAK NTVYLQMNSLKPEDTGIYYCAADMWADCGSMEYDYWG QGTQVTVSS |
| 17 | QVQLVESGGGLVQPGGSLRLSCAASGSILNLMTTGWYR QAPGKQRDLVATITFGGTTNYADSVKGRFTISRDKSKNT AYLQMDNLKPEDTAVYFCYADVIVGFQDRRVAWDAGTQ VTVSS |
| 18 | QVQLVESGGGLVQPGGSLRLSCAASGSILNLMTTGWYR QAPGKQRDLVATITFGGTTNYADAVKGRFTISRDNAKNT VYLQMNNLEPEDTAVYFCYLDYIFGFGDRRVAWDQGTQ VTVSS |
| 19 | EVQLVESGGGLVQPGGSLRLSCAASGNILSINYAGWYR QAPGKERELVALITRGGSTTYGASLKGRFTISSDNAKDT VYLQMSSLQPDDTAVYYCNFRINFGILGGLRDYWGQGT QVTVSP |
| 20 | QVQLVESGGGLVQPGGSLRLSCAASGNILSINYMGWYR QAPGKERELVALITRGGTTNYAESLKGRFTISGDNAKDT VYLQMNSLQPEDTAVYYCYFRINFGILGGLRDYWGQGT QVTVSS |
| 21 | QVQLVESGGGLVQTGGSLRLSCAASDSTFSSYIMGWVR QAPGKGLEWVAGITWSGGIKDYADSVKGRFTIARDNAK NTVYLQMNSLKPEDAAIYYCAANAIQLTRRSGDYAYWG QGTQVTVSS |
| 22 | QVQLVESGGGLVQPGGSLRLSCAASDSTFSSYIMGWFR QAPGKGLEFVAGITWSGGIKDYADSVKGRFTIARDNSKN TLYLQMNSLRAEDAAVYYCAANAIQLTRRSGDYAYWGQ GTLVTVSS |
| 23 | QVQLVESGGGLVQPGGSLRLSCAASDSTFSSYIMGWFR QAPGKGLEFVAGITWSGGIKDYADSVKGRFTIARDNAKN TLYLQMNSLRAEDAAVYYCAANAIQLTRRSGDYAYWGQ GTQVTVSS |
| 24 | QVQLVESGGGLVQTGGSLRLSCAASDSTFSSYIMGWFR QAPGKGLEWVAGITWSGGIKDYADSVKGRFTIARDNAK NTVYLQMNSLKPEDAAIYYCAANAIQLTRRSGDYAYWG QGTQVTVSS |
| 25 | QVQLVESGGGLVQPGGSLRLSCAASGSILNLMTTGWYR QAPGKGLDLVATITFGGTTNYADAVKGRFTISRDNSKNTL YLQMNSLRAEDTAVYFCYLDYIFGFGDRRVAWDQGTLVT VSS |
| 26 | EVQLVESGGGLVQPGGSLRLSCAASGNILSINYMGWYR QAPGKGLELVATITRGGSTNYQGNLKGRFIISGDNSKDTL |
| | YLQMNSLRAEDTAVYYCYFRINFGILGGLRDYWGQGTLV TVSS |

**Table 9 CAR composition sequence**

| SEQ ID. NO. | Structure | Sequence |
|---|---|---|
| 27 | CD8 signal peptide | MALPVTALLLPLALLLHAARP |
| 28 | Hinge+flag tag | TTTDYKDDDDKPAPRPPTPAPTIASQPLSLRPEACRPAAG GAVHTRGLDFACD |
| 29 | CD28 transmembrane region | FWVLVVVGGVLACYSLLVTVAFIIFWV |
| 30 | CD28 intracellular region | RSKRSRLLHSDYMNMTPRRPGPTRKHYQPYAPPRDFAA YRS |
| 31 | CD3 ζ intracellular region | RVKFSRSADAPAYQQGQNQLYNELNLGRREEYDVLDKR RGRDPEMGGKPRRKNPQEGLYNELQKDKMAEAYSEIGM KGERRRGKGHDGLYQGLSTATKDTYDALHMQALPPR |
| 32 | 4-1BB co-stimulatory signal intracellular region | RFSVVKRGRKKLLYIFKQPFMRPVQTTQEEDGCSCRFPE EEEGGCELR |
| 33 | CAR02 | MALPVTALLLPLALLLHAARPEVQLVESGGGLVQPGGSLR LSCAASGNILSINYMGWYRQAPGKERELVATITRGGSTNY QGNLKGRFIISGDNTKDTVYLQMNNLQPEDTAVYYCYFRI NFGILGGLRDYWGQGTQVTVSSTTTDYKDDDDKPAPRPP TPAPTIASQPLSLRPEACRPAAGGAVHTRGLDFACDFWVL VVVGGVLACYSLLVTVAFIIFWVRSKRSRLLHSDYMNMTP RRPGPTRKHYQPYAPPRDFAAYRSRVKFSRSADAPAYQQ GQNQLYNELNLGRREEYDVLDKRRGRDPEMGGKPRRKN PQEGLYNELQKDKMAEAYSEIGMKGERRRGKGHDGLYQ GLSTATKDTYDALHMQALPPR |
| 34 | CAR05 | MALPVTALLLPLALLLHAARPQVQLVESGGGLVQTGGSLR LSCAASDSTFSSYIMGWFRQAPGKEREFVAGITWSGGIK DYADSVKGRFTIARDNAKNTVYLQMNSLKPEDAAIYYCAA NAIQLTRRSGDYAYWGQGTQVTVSSTTTDYKDDDDKPAP RPPTPAPTIASQPLSLRPEACRPAAGGAVHTRGLDFACDF WVLVVVGGVLACYSLLVTVAFIIFWVRSKRSRLLHSDYMN MTPRRPGPTRKHYQPYAPPRDFAAYRSRVKFSRSADAPA YQQGQNQLYNELNLGRREEYDVLDKRRGRDPEMGGKP RRKNPQEGLYNELQKDKMAEAYSEIGMKGERRRGKGHD GLYQGLSTATKDTYDALHMQALPPR |
| 35 | CAR18 | MALPVTALLLPLALLLHAARPQVQLVESGGGLVQPGGSLR LSCAASGSILNLMTTGWYRQAPGKQRDLVATITFGGTTNY ADAVKGRFTISRDNAKNTVYLQMNNLEPEDTAVYFCYLDY IFGFGDRRVAWDQGTQVTVSSTTTDYKDDDDKPAPRPPT PAPTIASQPLSLRPEACRPAAGGAVHTRGLDFACDFWVLV VVGGVLACYSLLVTVAFIIFWVRSKRSRLLHSDYMNMTPR RPGPTRKHYQPYAPPRDFAAYRSRVKFSRSADAPAYQQG QNQLYNELNLGRREEYDVLDKRRGRDPEMGGKPRRKNP QEGLYNELQKDKMAEAYSEIGMKGERRRGKGHDGLYQG LSTATKDTYDALHMQALPPR |

All documents mentioned herein are incorporated by reference into the present application as if each document were individually incorporated by reference. In addition, it should be understood that, after reading the above teachings of the present invention, those skilled in the art may make various changes or modifications to the present invention, and these equivalent forms also fall within the scope defined by the claims attached to the present application.

## Claims

1. A nanobody that specifically binds to Claudin18.2, wherein complementary determining regions, or CDR regions, of a VHH chain of the nanobody are selected from the group consisting of:
(1) CDR1 as shown in SEQ ID NO: 36, CDR2 as shown in SEQ ID NO: 37, and CDR3 as shown in SEQ ID NO: 38;
(2) CDR1 as shown in SEQ ID NO: 39, CDR2 as shown in SEQ ID NO: 40, and CDR3 as shown in SEQ ID NO: 41;
(3) CDR1 as shown in SEQ ID NO: 42, CDR2 as shown in SEQ ID NO: 43, and CDR3 as shown in SEQ ID NO: 44;
(4) CDR1 as shown in SEQ ID NO: 45, CDR2 as shown in SEQ ID NO: 46, and CDR3 as shown in SEQ ID NO: 47;
(5) CDR1 as shown in SEQ ID NO: 48, CDR2 as shown in SEQ ID NO: 49, and CDR3 as shown in SEQ ID NO: 50;
(6) CDR1 as shown in SEQ ID NO: 51, CDR2 as shown in SEQ ID NO: 52, and CDR3 as shown in SEQ ID NO: 53;
(7) CDR1 as shown in SEQ ID NO: 39, CDR2 as shown in SEQ ID NO: 54, and CDR3 as shown in SEQ ID NO: 41;
(8) CDR1 as shown in SEQ ID NO: 39, CDR2 as shown in SEQ ID NO: 55, and CDR3 as shown in SEQ ID NO: 41;
(9) CDR1 as shown in SEQ ID NO: 67, CDR2 as shown in SEQ ID NO: 68, and CDR3 as shown in SEQ ID NO: 41;
(10) CDR1 as shown in SEQ ID NO: 39, CDR2 as shown in SEQ ID NO: 69, and CDR3 as shown in SEQ ID NO: 41; and/or
(11) CDR1 as shown in SEQ ID NO: 36, CDR2 as shown in SEQ ID NO: 65, and CDR3 as shown in SEQ ID NO: 66.

2. The nanobody according to claim 1, wherein CDR1, CDR2, and CDR3 are separated by framework regions FR1, FR2, FR3, and FR4 of the VHH chain.

3. The nanobody according to claim 1, wherein the amino acid sequence of the VHH chain of the nanobody is selected from the group consisting of: SEQ ID NOs: 1-26, or combinations thereof.

4. A multivalent antibody or multispecific antibody that specifically binds to Claudin18.2, wherein the multivalent antibody or multispecific antibody comprises at least one antibody component that targets a Claudin18.2 epitope, the antibody component being the nanobody according to claim 1.

5. The multivalent antibody or multispecific antibody according to claim 4, wherein the anti-Claudin18.2 antibody comprises one or more VHH chains having the amino acid sequence as shown in any one of SEQ ID NOs: 1-26.

6. A recombinant protein, wherein the recombinant protein comprises:
(i) the nanobody that specifically binds to Claudin18.2 according to claim 1, or the multivalent antibody or multispecific antibody that specifically binds to Claudin18.2 according to claim 4; and
(ii) optionally a tag sequence that aids in expression and/or purification.

7. A chimeric antigen receptor (CAR) fusion protein, wherein the chimeric antigen receptor (CAR) fusion protein comprises, from N-terminus to C-terminus:
(i) an antigen-binding domain that specifically binds to Claudin18.2, the antigen-binding domain comprising the nanobody according to claim 1;
(ii) a transmembrane domain;
(iii) at least one co-stimulatory domain; and
(iv) an activation domain.

8. The chimeric antigen receptor (CAR) fusion protein according to claim 7, wherein the CAR has a structure as shown in Formula Ia:
L-VHH-H-TM-C-CD3ζ (la)
where,
each "-" is independently a linker peptide or peptide bond;
L is a signal peptide sequence;
VHH is an antigen-binding domain that specifically binds to Claudin18.2;
H is a hinge region;
TM is a transmembrane domain;
C is a co-stimulatory signal domain; and
CD3ζ is a cytoplasmic signal transduction sequence (including wild-type, or mutant/modification thereof) derived from CD3ζ.

9. The chimeric antigen receptor (CAR) fusion protein according to claim 8, wherein the amino acid sequence of the VHH is as shown in SEQ ID NO: 2, 5, or 18.

10. An antibody-drug conjugate, wherein the antibody-drug conjugate comprises:
(a) the nanobody according to claim 1, the multivalent antibody or multispecific antibody according to claim 4, or the recombinant protein according to claim 6; and
(b) a conjugating moiety that is conjugated to the antibody moiety, the conjugating moiety being selected from the group consisting of: detectable labels, drugs, toxins, cytokines, radionuclides, enzymes, or combinations thereof.

11. A polynucleotide, wherein the polynucleotide encodes a protein selected from the group consisting of: the nanobody according to claim 1, the multivalent antibody or multispecific antibody according to claim 4, the recombinant protein according to claim 6, or the CAR fusion protein according to claim 7.

12. An expression vector, wherein the expression vector comprises the polynucleotide according to claim 11.

13. A host cell, wherein the host cell comprises the expression vector according to claim 12, or having the polynucleotide according to claim 11 integrated in a genome thereof, or expressing the nanobody according to claim 1, the multivalent antibody or multispecific antibody according to claim 4, the recombinant protein according to claim 6, or the CAR fusion protein according to claim 7.

14. A pharmaceutical composition, wherein the pharmaceutical composition comprises:
(i) the nanobody according to claim 1, the multivalent antibody or multispecific antibody according to claim 4, the recombinant protein according to claim 6, or an immune cell that expresses the CAR fusion protein according to claim 7; and
(ii) a pharmaceutically acceptable carrier.

15. A use of the nanobody according to claim 1, the multivalent antibody or multispecific antibody according to claim 4, the recombinant protein according to claim 6, or an immune cell that expresses the CAR fusion protein according to claim 7, wherein the use is in the preparation of a drug for the prevention and/or treatment of a cancer or tumor related to Claudin18.2.
